# EUROPEAN PATENT APPLICATION

(11) **EP 2 243 830 A1**
(43) Date of publication of application: **27.10.2010**
(21) Application number: 09710080.4
(22) Date of filing: 10.02.2009
(51) Int. Cl.: C12N 15/09, C12Q 1/02, C12Q 1/48, A61K 45/00, A61P 25/00, A61P 25/28, G01N 33/15, G01N 33/50

(54) **SCREENING METHOD**

(30) Priority: 12.02.2008 JP 2008030946
(71) Applicant: Takeda Pharmaceutical Company Limited, Osaka 541-0045 (JP)
(72) Inventor: NOGUCHI, Kenichi, Osaka-shi Osaka 532-8686 (JP); HORIGUCHI, Takashi, Osaka-shi Osaka 532-8686 (JP)
(74) Representative: von Kreisler, Alek
(86) International application number: PCT/JP2009/052235
(87) International publication number: WO 2009/101942

(57) **Abstract**

The present invention provides a cell model system capable of sufficiently reproducing a series of tau lesions, and provides a screening method for a prophylactic or therapeutic drug for a neurodegenerative disease using phosphorylation, insolubilization or aggregation of tau, or neurite denaturation or cell death in the cell model as an index.

## Description

### Technical Field

The present invention relates to a novel screening method for a prophylactic and/or therapeutic drug for a neurodegenerative disease.

### (Background of the Invention)

Neurofibrillary tangle is a major lesion of Alzheimer's disease (AD), and is comparable to senile plaque accumulation. The spread thereof is used as a scale showing the progression of AD, and correlates well with the decline of cognitive function. Neurofibrillary tangle is constituted by fibers with a helical structure not found in normal cells, which are called paired helical filaments (PHF) or straight filaments (SF). The main component of PHF and SF is a microtubule-binding protein tau and is highly phosphorylated to have a β sheet structure and accumulated. Neurodegenerative diseases associated with abnormal accumulation of tau are called taupathies, and includes, besides AD, Pick disease, corticobasal degeneration, progressive supranuclear palsy and the like. It has been found that a family affected with familial frontotemporal dementia called frontotemporal dementia with Parkinsonism linked to chromosome 17 (FTDP-17) included in Pick disease has tau gene mutation as etiology. As mentioned above, there is a close relationship between abnormal accumulation of tau and neurodegeneration.

Tau is an extremely highly soluble protein having, on its C-terminal side, a microtubule-binding site with a 3 or 4 repeat structure caused by splicing, and is considered to be bound to and involved in the stabilization of the microtubule. The microtubule stabilizing ability of tau is considered to be controlled by as many as 20 phosphorylation sites in tau. In fact, phosphorylation occurs in many sites during the fetal stage when construction of neural network system is active. However, phosphorylation is hardly observed in the adulthood.

Different from the healthy individual, tau in the brain of AD patients is highly phosphorylated, has lost microtubule-binding capacity, and is accumulated, aggregated and insolubilized in the cell body. Tau in AD patients contains a specific phosphorylation site not found in the fetal stage. It has been reported that an AT-100 antibody specifically recognizes tau in AD patients, and the recognition sites thereof include phosphorylated 214th serine residue (S214). These facts indicate that phosphorylation and aggregation of tau, and decline of neurological function are closely connected, and an improvement of tau lesion leads to the prophylaxis or treatment of neurodegenerative diseases such as AD and the like.

As a kinase that phosphorylates S214, protein kinase A (cAMP dependent serine/threonine kinase, PKA) has been reported. PKA has been reported to possibly phosphorylate S214 not only in vitro but also in vivo (non-patent document 1). Of the subtypes of PKA, PKAβ has been reported to colocalize with neurofibrillary tangle (NFT), thus suggesting a close relationship thereof with the progression of tau lesion (non-patent document 2).

Protein kinase X linked (PRKX) is a cAMP dependent serine/threonine kinase present on the X chromosome. Except the sister gene PRKY present on the Y chromosome, PRKX shows the highest homology to PKA, and a reported PKA inhibitor H-89 is shown to also inhibit PRKX (non-patent document 3). Recently, a report has documented that PRKX expression increases along with the progression of AD, and is related to the phosphorylation of tau and nerve cell death (patent document 1).

An evaluation system using the cell is an effective means for the search and evaluation of drug discovery targets and compounds. For the development of a therapeutic drug for neurodegenerative diseases such as AD and the like, a cell model that reproduces tau lesions such as phosphorylation of tau and subsequent aggregation and cell death is extremely useful since candidate compounds can be searched and evaluated more directly using the improvement of such lesions as an index. However, a cell model system capable of sufficiently reproducing a series of tau lesions to the extent permitting use of improvement of tau lesion as an index has not been obtained.
patent document 1: WO2006/128879
non-patent document 1: FEBS Lett., vol. 579, page 251-8, 2005
non-patent document 2: J. Neurosci., vol. 19, page 7486-94, 1999
non-patent document 3: Proc. Natl. Acad. Sci. USA, vol. 99, page 9260-5, 2002

### Disclosure of the Invention

### Problems to be Solved by the Invention

Therefore, an object of the present invention is to provide a cell model system capable of sufficiently reproducing a series of tau lesions, and a screening method for a prophylactic or therapeutic drug for a neurodegenerative disease, using, as an index, phosphorylation, insolubilization or aggregation of tau, or neurite denaturation or cell death in the cell model.

### Means of Solving the Problems

The present inventors have conducted intensive studies in an attempt to solve the above-mentioned problems and found that introduction of a PRKX gene into a tau overexpression cell line or primary nerve cell can induce, in the cell, phosphorylation of tau and subsequent clear insolubilization and aggregation of tau, as well as cell death. Furthermore, they have succeeded in constructing a screening system for a prophylactic or therapeutic substance for a neurodegenerative disease using the cell. The present inventors conducted further investigations based on these findings, and developed the present invention.

Accordingly, the present invention provides
[1] a method of screening for a prophylactic or therapeutic substance for a neurodegenerative disease, comprising contacting a test substance with a cell overexpressing a PRKX gene and a tau gene, which incorporates the PRKX and tau genes, and detecting phosphorylation, insolubilization or aggregation of tau in the cell,
[2] the method according to the above-mentioned [1], wherein the phosphorylation of tau is phosphorylation of the 214-position Ser,
[3] the method according to the above-mentioned [1], further comprising measuring the amount of detergent-insoluble tau,
[4] the method according to the above-mentioned [1], further comprising counting cells positive to a probe recognizing a β sheet structure,
[5] the method according to the above-mentioned [1], wherein the cell is an established cell line,
[6] the method according to the above-mentioned [1], wherein the cell is a H4 neuroglial cell,
[7] a method of screening for a prophylactic or therapeutic substance for a neurodegenerative disease, comprising contacting a test substance with a primary nerve cell incorporating a PRKX gene and overexpressing the PRKX gene and detecting, in the cell, phosphorylation, insolubilization or aggregation of tau, neurite denaturation or cell death,
[8] the method according to the above-mentioned [1] or [7], wherein the PRKX gene is introduced by a virus vector,
[9] the method according to the above-mentioned [8], wherein the virus vector is lentivirus,
[10] a method of screening for a prophylactic or therapeutic substance for a neurodegenerative disease, comprising contacting a test substance with a PRKX protein and a tau protein, and detecting phosphorylation, insolubilization or aggregation of tau,
[11] the method according to the above-mentioned [1], [7] or [10], wherein the neurodegenerative disease is a disease involving a tau lesion,
[12] the method according to the above-mentioned [11], wherein the disease involving a tau lesion is Alzheimer's disease, and the like.

### Effect of the Invention

The present invention characteristically uses, as an evaluation system, a cell model capable of sufficiently reproducing a series of tau lesions, which is obtained by introduction of a PRKX gene into a tau overexpression cell line or primary nerve cell, thus enabling conventionally unavailable search and evaluation of a prophylactic or therapeutic drug for a neurodegenerative disease using a tau lesion improvement effect as an index.

### (Detailed Description of the Invention)

In the present specification, the "overexpression" means that the expression level of a particular gene in a certain cell is, for example, not less than 10-fold that of a naturally occurring endogenous gene thereof.

### (1) Screening using a cell overexpressing a PRKX gene and a tau gene, which incorporates the PRKX and tau genes

The present invention provides a method of screening for a prophylactic or therapeutic substance for a neurodegenerative disease, comprising contacting a test substance with a cell overexpressing a PRKX gene and a tau gene, which incorporates said genes, and detecting phosphorylation, insolubilization or aggregation of tau in the cell.

The "cell overexpressing a PRKX gene and a tau gene" to be used in the present invention is not particularly limited as long as it permits introduction of a nucleic acid encoding PRKX or a partial peptide thereof and a nucleic acid encoding tau or a partial peptide thereof, and sufficient observation of phosphorylation, insolubilization and aggregation of tau to the degree efficacy of a test substance can be evaluated with improvement of the tau lesion as an index (e.g., cell with expression level of, for example, not less than 10-fold that of a naturally occurring endogenous gene thereof).

The PRKX in the present invention is a protein comprising the same or substantially the same amino acid sequence as the amino acid sequence shown by SEQ ID NO: 2. Moreover, tau in the present invention is a protein comprising the same or substantially the same amino acid sequence as the amino acid sequence shown by SEQ ID NO:4. In the present description, proteins and peptides are denoted with the N-terminus (amino terminus) described as the left end and the C-terminus (carboxyl terminus) as the right end, in accordance with the common way of describing peptides.

As the "substantially the same amino acid sequence as the amino acid sequence shown by SEQ ID NO:2 (or SEQ ID NO:4)", an amino acid sequence having a similarity of about 70% or more, preferably about 80% or more, more preferably about 90% or more, still more preferably about 95% or more, particularly preferably about 97% or more, and most preferably about 98% or more, to the amino acid sequence shown by SEQ ID NO:2 (or SEQ ID NO:4) and the like can be mentioned. Here, the "similarity" means a ratio (%) of identical amino acid residues and similar amino acid residues to all overlapping amino acid residues in the best alignment where two amino acid sequences are aligned using a mathematical algorithm known in the technical field (preferably, the algorithm considers introduction of gaps on one or both sides of the sequence for the best alignment). "A similar amino acid" means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such similar amino acids is expected to give no change in the phenotype of protein (i.e., constitutive amino acid substitution). Specific examples of constitutive amino acid substitution are obvious in the relevant technical field, and are described in various documents (see, for example, Bowie et al., Science, 247:1306-1310 (1990)).

The similarity of the amino acid sequences in the present specification can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool). As other algorithm for determining the similarity of amino acid sequence, for example, the algorithm described in Karlin et al., Proc. Natl. Acad. Sci. USA, 90: 5873-5877 (1993) [the algorithm is incorporated in NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in GCG software package], the algorithm described in Myers and Miller, CABIOS, 4: 11-17 (1988) [the algorithm is incorporated in ALIGN program (version 2.0) which is a part of the CGC sequence alignment software package], the algorithm described in Pearson et al., Proc. Natl. Acad. Sci. USA, 85: 2444-2448 (1988) [the algorithm is incorporated in the FASTA program in GCG software package] and the like can be mentioned, and they can be preferably used in a similar way.

More preferably, as substantially the same amino acid sequence as the amino acid sequence shown by SEQ ID NO:2 (or SEQ ID NO:4), an amino acid sequence having a homology of about 70% or more, preferably about 80% or more, more preferably about 90% or more, still more preferably about 95% or more, particularly preferably about 97% or more, and most preferably about 98% or more, to the amino acid sequence shown by SEQ ID NO:2 (or SEQ ID NO:4), can be mentioned.

The "protein having the same or substantially the same amino acid sequence as the amino acid sequence shown by SEQ ID NO:2 (or SEQ ID NO:4)" is a protein containing an amino acid sequence substantially the same as the amino acid sequence shown by SEQ ID NO:2 (or SEQ ID NO:4), and substantially the same quality of activity as that of a protein consisting of the amino acid sequence shown by SEQ ID NO:2 (or SEQ ID NO:4).
The "activity" here means serine/threonine kinase activity (particularly tau phosphorylation activity), tau insolubilization, aggregation induction activity, cell death induction activity and the like for PRKX, and an activity to, upon high phosphorylation, be intracellularly accumulated, aggregated and insolubilized to induce cell death and the like for tau. In addition, the "substantially the same quality" means, for example, that the properties are physiologically or pharmacologically the same by qualification. Therefore, PRKX and tau activities are preferably equal, but quantitative elements such as the level of these activities (e.g., about 0.01- to about 100-fold, preferably about 0.1- to about 10-fold, more preferably 0.5- to 2-fold), molecular weight of protein and the like may be different.
The PRKX and tau activities can be measured according to a method known per se and, for example, according to the method described in the Examples to be described later and the like.

Examples of the PRKX of the present invention also include what is called muteins of proteins comprising
(i) an amino acid sequence having one or two or more amino acids (e.g., about 1 to about 50, preferably about 1 to about 30, more preferably about 1 to about 10, still more preferably several (1 to 5, 4, 3 or 2) amino acids) deleted from the amino acid sequence shown by SEQ ID NO:2,
(ii) an amino acid sequence having one or two or more amino acids (e.g., about 1 to about 50, preferably about 1 to about 30, more preferably about 1 to about 10, still more preferably several (1 to 5, 4, 3 or 2) amino acids) added to the amino acid sequence shown by SEQ ID NO:2,
(iii) an amino acid sequence having one or two or more amino acid (e.g., about 1 to about 50, preferably about 1 to about 30, more preferably about 1 to about 10, still more preferably several (1 to 5, 4, 3 or 2) amino acids) inserted to the amino acid sequence shown by SEQ ID NO:2,
(iv) an amino acid sequence having one or two or more amino acids (e.g., about 1 to about 50, preferably about 1 to about 30, more preferably about 1 to about 10, still more preferably several (1 to 5, 4, 3 or 2) amino acids) substituted with other amino acids in the amino acid sequence shown by SEQ ID NO:2, or
(v) an amino acid sequence comprising a combination thereof. Similarly, examples of the tau of the present invention also include proteins comprising (i) an amino acid sequence having one or two or more amino acids (e.g., about 1 to about 100, preferably about 1 to about 50, more preferably about 1 to about 10, still more preferably several (1 to 5, 4, 3 or 2) amino acids) deleted from the amino acid sequence shown by SEQ ID NO:4,
(ii) an amino acid sequence having one or two or more amino acids (e.g., about 1 to about 100, preferably about 1 to about 50, more preferably about 1 to about 10, still more preferably several (1 to 5, 4, 3 or 2) amino acids) added to the amino acid sequence shown by SEQ ID NO:4,
(iii) an amino acid sequence having one or two or more amino acid (e.g., about 1 to about 100, preferably about 1 to about 50, more preferably about 1 to about 10, still more preferably several (1 to 5, 4, 3 or 2) amino acids) inserted to the amino acid sequence shown by SEQ ID NO:4,
(iv) an amino acid sequence having one or two or more amino acids (e.g., about 1 to about 100, preferably about 1 to about 50, more preferably about 1 to about 10, still more preferably several (1 to 5, 4, 3 or 2) amino acids) substituted with other amino acids in the amino acid sequence shown by SEQ ID NO:4, or
(v) an amino acid sequence comprising a combination thereof.
When an amino acid sequence is inserted, deleted or substituted as described above, the position of the insertion, deletion or substitution is not subject to limitation, as long as the protein retains the above-mentioned PRKX and tau activities.

Preferable examples of the PRKX protein include human PRKX consisting of the amino acid sequence shown by SEQ ID NO:2 (RefSeq Accession No. NP_005035.1), orthologs thereof in other mammals (e.g., mouse ortholog registered in GenBank as Accession No. NP_058675.1, rat ortholog registered as Accession No. NP_001029135.1, chimpanzee ortholog registered as Accession No. XP_001141503.1, dog ortholog registered as Accession No. XP_852513.1 etc.), naturally occurring allele variants thereof and the like. Preferable examples of the tau protein include human tau consisting of the amino acid sequence shown by SEQ ID NO:4 (441 amino acids) (RefSeq Accession No. NP_005901.2) and isoforms thereof (in the amino acid sequence shown by SEQ ID NO:4, an amino acid sequence consisting of 352 amino acids and defective in the amino acid sequence shown by amino acid Nos. 45-102 and 275-305, an amino acid sequence consisting of 383 amino acids and defective in the amino acid sequence shown by amino acid Nos. 45-102, an amino acid sequence consisting of 381 amino acids and defective in the amino acid sequence shown by amino acid Nos. 74-102 and 275-305, an amino acid sequence consisting of 412 amino acids and defective in the amino acid sequence shown by amino acid Nos. 74-102, and an amino acid sequence consisting of 410 amino acids and defective in the amino acid sequence shown by amino acid Nos. 275-305), orthologs thereof in other mammals (e.g., mouse ortholog registered in GenBank as Accession No. NP_001033698.1, rat ortholog registered as Accession No. NP_058908.2 etc.), naturally occurring allele variants thereof and the like.

The partial peptides of PRKX and tau in the present invention are peptides having a partial amino acid sequence of the above-mentioned PRKX and tau proteins, and an activity substantially the same as PRKX. As used herein, the "substantially the same quality of activity" is as defined above. In addition, "substantially the same quality of activity" can be measured in the same manner as mentioned above.
The partial peptide of PRKX is not particularly limited as long as it has the above-mentioned properties, and specifically includes, for example, a peptide containing a protein kinase domain shown by amino acid Nos. 46-300 in the amino acid sequence shown by SEQ ID NO:2 (or region corresponding to PRKX ortholog in non-human mammal) and the like.
The partial peptide of tau is not particularly limited as long as it has the above-mentioned properties, and specifically includes, for example, a peptide containing an amino acid sequence shown by amino acid Nos. 198-262 and 396-422, which includes a highly phosphorylated site found in PHF tau, in the amino acid sequence shown by SEQ ID NO:4 and the like.

The nucleic acid encoding PRKX or a partial peptide thereof is not particularly limited as long as it encode a protein containing an amino acid sequence the same or substantially the same as the aforementioned amino acid sequence shown by SEQ ID NO:2 or a partial peptide thereof, and specifically includes, for example, a nucleic acid containing the nucleotide sequence shown by SEQ ID NO:1, a nucleic acid containing a nucleotide sequence that hybridizes with the nucleotide sequence shown by SEQ ID NO:1 under high stringent conditions, and encoding a protein or peptide having substantially the same quality of activity (e.g., serine/threonine kinase activity (particularly tau phosphorylation activity), tau insolubilization, aggregation and induction activity, cell death induction activity and the like) as a protein containing the amino acid sequence shown by SEQ ID NO:2 and the like. The nucleic acid may be DNA or RNA, preferably DNA.

The nucleic acid encoding tau or a partial peptide thereof is not particularly limited as long as it encodes a protein containing the same or substantially the same amino acid sequence as the aforementioned amino acid sequence shown by SEQ ID NO:4 or a partial peptide thereof, and includes, for example, a nucleic acid containing the nucleotide sequence shown by SEQ ID NO:3, a nucleic acid containing a nucleotide sequence that hybridizes with the nucleotide sequence shown by SEQ ID NO:3 under high stringent conditions, and encoding a protein or peptide having substantially the same quality of activity (e.g., an activity to, upon high phosphorylation, be intracellularly accumulated, aggregated and insolubilized to induce cell death and the like) as a protein containing the amino acid sequence shown by SEQ ID NO:4 and the like. The nucleic acid may be DNA or RNA, preferably DNA.

As the nucleic acid capable of hybridizing with the nucleotide sequence shown by SEQ ID NO:1 or SEQ ID NO:3 under high stringent conditions, for example, a nucleic acid containing a nucleotide sequence having a similarity of about 70% or more, preferably about 80% or more, more preferably about 90% or more, still more preferably about 95% or more, particularly preferably about 97% or more, and most preferably about 98% or more, to the nucleotide sequence shown by SEQ ID NO:1 or SEQ ID NO:3 and the like is used.
The similarity of the base sequences in the present specification can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool). As other algorithm with which to determine the similarity of the base sequence, the homology calculation algorithm of the above-mentioned amino acid sequence can be preferably used in the same manner.

The hybridization can be performed by a method known per se or a method analogous thereto, for example, a method described in Molecular Cloning, 2nd ed. (J. Sambrook et al., Cold Spring Harbor Lab. Press, 1989) and the like. A commercially available library can also be used according to the instructions of the attached manufacturer's protocol.
"High-stringent conditions" refer to, for example, conditions involving a sodium salt concentration of about 19 to about 40 mM, preferably about 19 to about 20 mM, and a temperature of about 50 to about 70°C, preferably about 60 to about 65°C. In particular, a case wherein the sodium concentration is about 19 mM and the temperature is about 65°C is preferred.

The DNA encoding PRKX is preferably a DNA containing a nucleotide sequence encoding a human PRKX protein consisting of the nucleotide sequence shown by SEQ ID NO:1, or an ortholog thereof in other mammal. The DNA encoding tau is preferably a DNA containing a nucleotide sequence encoding a human tau protein (441 amino acids) consisting of the nucleotide sequence shown by SEQ ID NO:3, or an ortholog thereof in other mammal.

The DNA encoding PRKX or a partial peptide thereof and tau or a partial peptide thereof can be prepared from genome DNA, genome DNA library, or cDNA or cDNA library derived from any cell (e.g., hepatocyte, splenocyte, nerve cell, glial cell, pancreatic β cell, myeloid cell, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding progenitor cell, stem cell or cancer cell thereof, and the like) of a mammal (e.g., human, bovine, monkey, horse, swine, sheep, goat, dog, cat, guinea pig, rat, mouse, rabbit, hamster and the like), or any tissue where such cells are present (e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle and the like). That is, directly amplification is possible by Polymerase Chain Reaction (PCR) method and Reverse Transcriptase-PCR (RT-PCR) method and using the genome DNA fraction and total RNA or mRNA fraction prepared from the above-mentioned cells or tissues each as a template. Alternatively, the DNA can also be cloned by colony or plaque hybridization method, PCR method and the like, from the genomic DNA library or cDNA library prepared by inserting, into a suitable vector, a fragment of genomic DNA and whole RNA or mRNA prepared from the above-mentioned cell/tissue. The vector to be used for the library may be any of bacteriophage, plasmid, cosmid, phagemid and the like.
In addition, DNA encoding PRKX or a partial peptide thereof and tau or a partial peptide thereof can also be chemically synthesized based on, for example, the nucleotide sequence shown by SEQ ID NO: 1 or SEQ ID NO:3 and by a known method.

The base sequence of the DNA can be converted according to a method known per se, such as the ODA-LA PCR method, the Gapped duplex method, or the Kunkel method, or a method based thereon, using a commonly known kit, for example, Mutan^{™}-super Express Km (TAKARA SHUZO CO. LTD.), Mutan^{™}-K (TAKARA SHUZO CO. LTD.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added by using a suitable synthetic DNA adaptor.

A host cell to be used for the preparation of the cell overexpressing a PRKX gene and a tau gene in the present invention is not particularly limited as long as it can express PRKX and tau in excess (i.e., amount sufficient to cause phosphorylation, insolubilization and aggregation of tau to the degree efficacy of a test substance can be evaluated, e.g., expression level not less than 10-fold that of a naturally occurring endogenous gene thereof) when a nucleic acid encoding the above-mentioned PRKX or a partial peptide thereof (hereinafter sometimes to be simply referred to as "PRKX") and a nucleic acid encoding tau or a partial peptide thereof (hereinafter sometimes to be simply referred to as "tau") is introduced. Preferred are various established cells such as mammalian cells, for example, human nerve cell line (e.g., SH-SY5Y, NB-1, PC12, NT-2, IMR-32), human nuroglia cell line (e.g., H4), HepG2 cell, HEK293 cell, HeLa cell, human FL cell, monkeyCOS-7 cell, monkey Vero cell, Chinese hamster ovary (CHO) cell, dhfr gene defective CHO cell, mouse L cell, mouse AtT-20 cell, mouse myeloma cell, rat H4IIE-C3 cell, rat GH3 cell and the like. Among these, H4 nuroglia cell is preferable.

DNA encoding PRKX and tau is preferably inserted into an expression vector compatible with the above-mentioned host cell and introduced into the host cell. As the expression vector, animal cell expression plasmid (e.g., pA1-11, pXT1, pRc/CMV, pRc/RSV, pcDNAI/Neo); bacteriophage such as λ phage and the like; animal virus vector such as lentivirus, retrovirus, vaccinia virus, adenovirus, adeno-associated virus and the like; and the like are used.
In one preferable embodiment of the present invention, a DNA encoding PRKX is introduced into a host cell using a virus vector, more preferably a lentivirus vector.

A promoter used for an expression vector may be any as long as it can function in a host cell. For example, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter and the like are used. Of these, CMV promoter, SRα promoter and the like are preferred.

Useful expression vectors include, in addition to the above, expression vectors that optionally comprise an enhancer, a splicing signal, a polyA addition signal, a selection marker, an SV40 replication origin (hereinafter also abbreviated as SV40ori), and the like. As examples of the selection markers, the dihydrofolate reductase (dhfr) gene [methotrexate (MTX) resistance], the ampicillin resistance gene, the neomycin resistance gene (G418 resistance), and the like can be mentioned. In particular, when a dhfr gene defective CHO cell is used and the dhfr gene is used as the selection marker, a target gene can also be selected using a thymidine-free medium.

When a DNA encoding PRKX and a DNA encoding tau are both introduced into a host animal cell, these DNAs may be inserted dicistronically on the same vector, or monocistronically inserted using an IRES sequence. Alternatively, these DNAs may be each inserted into individual expression vectors, and introduced into a host cell by cotransfection.

Gene transfer can be performed according to a calcium phosphate coprecipitation method, a PEG method, an electroporation method, a microinjection method, a lipofection method and the like. For example, the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973) can be used.

A cell overexpressing a PRKX gene and a tau gene obtained as mentioned above can be cultivated in a medium such as MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The pH of a medium is preferably about 6 - 8. Culture is generally performed at about 30 - 40°C, and aeration and stirring are added as necessary.

Examples of the test substance to be subjected to the screening method of the present invention include protein, peptide, antibody, non-peptide compound, synthetic compound, fermentation product, cell extract, plant extract, tissue extract, plasma and the like, and these substances may be novel or publicly known.

A test substance can be contacted with the above-mentioned cell by, for example, adding the test substance to the above-mentioned medium or various buffers (e.g., HEPES buffer, phosphate buffer, phosphoric acid buffered saline, Tris-HCl buffer, borate buffer, acetate buffer and the like) and incubating the cells for a given time. While the concentration of the test substance to be added varies depending on the kind of the compound (solubility, toxicity etc.), for example, it is appropriately selected from the range of about 0.1 nM - about 100 nM. An example of the incubation time is about 10 min - about 24 hr.

In the screening method, a prophylactic or therapeutic activity against a neurodegenerative disease can be measured using phosphorylation, insolubilization or aggregation of tau in the above-mentioned cells as an index.

When phosphorylation of tau is used as an index, a prophylactic or therapeutic activity of a test substance against a neurodegenerative disease can be measured by using an antibody that shows specific affinity to phosphorylated tau alone and does not show affinity to non-phosphorylated tau (phosphorylated tau-specific antibody) and according to various immunoassays known per se. While the phosphorylated tau-specific antibody is not particularly limited, one specifically recognizing a phosphorylation site specifically found in PHF tau (e.g., S214, 208-position serine (S208), 210-position serine (S210), 212-position threonine (T212), 403-position threonine (T403), 412-position serine (S412), 422-position serine (S422) etc.) is preferable, and a phosphorylated tau-specific antibody recognizing S214 is particularly preferable. A phosphorylated tau-specific antibody can be produced by using phosphorylated tau or a partial peptide thereof as an antigen, and a polyclonal or monoclonal antibody production technique known per se. In addition, various phosphorylated tau-specific antibody, phosphorylated tau detection ELISA kit and the like are commercially available (e.g., BioSource, Sigma-Aldrich and the like) .

As a specific screening method using phosphorylation of tau as an index, for example,
(i) a method comprising quantifying phosphorylated tau in a sample solution (solution dissolving the above-mentioned cells) by competitively reacting a phosphorylated tau-specific antibody with the sample solution and labeled phosphorylated tau or a partial peptide thereof, and detecting the labeled protein (peptide) bound to the antibody,
(ii) a method comprising quantifying phosphorylated tau in a sample solution by simultaneously or continuously reacting the sample solution with the anti-tau antibody insolubilized on a carrier and a labeled phosphorylated tau-specific antibody, and measuring the amount (activity) of the labeling agent insolubilized on the carrier, and the like can be mentioned.
As examples of the labeling agent used for the assay using a labeling substance, a radioisotope, an enzyme, a fluorescent substance, a luminescent substance and the like can be used. As examples of the radioisotope, [¹²⁵I], [¹³¹I], [³H], [¹⁴C] [³²P], [³³P], [³⁵S] and the like can be used. As the above-described enzyme, those that are stable and high in specific activity are preferred; for example, peroxidase, alkaline phosphatase, β-galactosidase, β-glucosidase, malate dehydrogenase and the like can be used. As examples of the fluorescent substance, fluorescamine, fluorescein isothiocyanate, cyanine fluorescence dye and the like can be used. As examples of the luminescent substance, luminol, luminol derivative, luciferin, lucigenin and the like can be used. Furthermore, a biotin-(strepto)avidin system can also be used for binding of an antibody or an antigen and a labeling agent.

The quantification of phosphorylated tau using the phosphorylated tau-specific antibody is not subject to limitation, and any method of measurement can be used, as long as it is a measurement method wherein the amount of antibody, antigen or antibody-antigen complex corresponding to the amount of antigen in the sample solution is detected by a chemical or physical means and is calculated from a standard curve generated using standard solutions containing known amounts of antigen. For example, nephelometry, the competitive method, the immunometric method and the sandwich method are preferably used; it is preferable, for example, in terms of sensitivity and specificity, to use the sandwich method described below.

In insolubilizing the antigen or antibody, physical adsorption may be used, and a chemical bond in common use to insolubilize or immobilize a protein or an enzyme or the like, may also be used. As the carrier, insoluble polysaccharides such as agarose, dextran and cellulose, synthetic resins such as polystyrene, polyacrylamide and silicone, glass and the like can be mentioned.

In the sandwich method, the amount of phosphorylated tau in a sample solution can be quantified by reacting the sample solution to an anti-tau antibody insolubilized (primary reaction) and further reacting to phosphorylated tau-specific antibody labeled (secondary reaction), and thereafter measuring the amount or activity of the labeling agent on the insolubilizing carrier. The primary reaction and the secondary reaction may be conducted in the reverse order, and may be conducted simultaneously or after a time lag. The labeling agent and the method of insolubilization can be based on those described above. Also, in the immunoassay by the sandwich method, the antibody used as the antibody for a solid phase or the antibody for labeling needs not always be one kind; a mixture of two kinds or more of antibodies may be used for the purposes of measurement sensitivity improvement and the like.

The phosphorylated tau-specific antibody can be used for a measurement system other than the sandwich method, for example, the competitive method, the immunometric method or nephelometry and the like.
In the competitive method, phosphorylated tau and labeled phosphorylated tau in a sample solution are competitively reacted with an antibody, a non-reacted labeled antigen (F) and a labeled antigen (B) bonded to the antibody are separated (B/F separation), the amount of label of B or F is measured to quantify phosphorylated tau in the sample solution. For this reaction method, a liquid phase method wherein a soluble antibody is used as the antibody, and polyethylene glycol and a secondary antibody against the aforementioned antibody (primary antibody) and the like are used for B/F separation, and a solid-phase method wherein a solid-phase antibody is used as a primary antibody (direct method), or a soluble primary antibody is used and a solid-phase antibody is used as a secondary antibody (indirect method) are employed.
In the immunometric method, phosphorylated tau in a sample solution and solid-phased phosphorylated tau are competitively reacted with a given amount of a labeled antibody, and the solid phase and the liquid phase are separated, or phosphorylated tau in a sample solution and an excess amount of a labeled antibody are reacted, solid-phased phosphorylated tau is added to bond a non-reacted labeled antibody to the solid phase, and the solid phase is separated from the liquid phase. Then, the label of either phase is measured and the amount of antigen in the sample solution is quantified.
Also, in nephelometry, the amount of insoluble precipitate resulting from an antigen-antibody reaction in the gel or in the solution is measured. Even when the amount of phosphorylated tau in the sample solution is small and only a small amount of precipitate is obtained, laser nephelometry, which utilizes laser scattering, and the like are preferably used.

Alternatively, the amount of phosphorylated tau can be measured by an immunostaining method comprising immobilizing the above-mentioned cells, and detecting phosphorylated tau in situ using a phosphorylated tau-specific antibody as a primary antibody and a labeled secondary antibody recognizing the antibody.

In applying these individual immunological measurement methods to the quantification method of the present invention, it is unnecessary to set special conditions, procedures and the like. Making ordinary technical considerations for those skilled in the art to the ordinary conditions and procedures in each method, a measurement system for phosphorylated tau can be constructed. For details of these general technical means, compendia, books and the like can be referred to.
For example, edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.
Using the phosphorylation tau-specific antibody as described above, the amount of phosphorylated tau in a cell can be quantified with high sensitivity.

For example, in the above-mentioned screening method, when phosphorylation of tau is inhibited by not less than about 20%, preferably not less than about 30%, more preferably not less than about 50%, in the presence of a test substance as compared to that in the absence of the test substance, the test substance can be employed as a candidate prophylactic or therapeutic substance for a neurodegenerative disease, particularly a disease involving tau lesion.

Specific examples of the screening method using insolubilization of tau as an index include a method comprising dissolving the above-mentioned cells in a buffer containing a surfactant (e.g., dodecylsodium sulfate (SDS), Triton X-100, Sarcosyl (N-lauroylsarcosine sodium), NP-40 (Nonidet P-40), Tween 20 etc.) at a concentration of 0.1 - 1%, applying the obtained insoluble fraction (precipitation) to SDS-polyacrylamide electrophoresis, and measuring the amount of tau in the insoluble fraction by Western blot analysis using an anti-tau antibody and the like. Alternatively, the amount of tau in the fraction may be measured by applying the dissolved insoluble fraction to the aforementioned various immunoassays (here, the antibody for detection may be a phosphorylated tau-specific antibody or a generally anti-tau antibody).

For example, in the above-mentioned screening method, when the amount of the surfactant insoluble tau decreased by not less than about 20%, preferably not less than about 30%, more preferably not less than about 50%, in the presence of a test substance as compared to that in the absence of the test substance, the test substance can be selected as a candidate prophylactic or therapeutic substance for a neurodegenerative disease, particularly a disease involving tau lesion.

Specific examples of the screening method using aggregation of tau as an index include a method comprising immobilizing the above-mentioned cells as in the immunostaining method using the above-mentioned phosphorylated tau-specific antibody, contacting same with a probe (e.g., thioflavin S, thioflavin T, congo red and the like) recognizing a β sheet structure, and counting the positive cells stained by the probe and the like.

For example, in the above-mentioned screening method, when the number of the probe positive cells decreased by not less than about 20%, preferably not less than about 30%, more preferably not less than about 50%, in the presence of a test substance as compared to that in the absence of the test substance, the test substance can be selected as a candidate prophylactic or therapeutic substance for a neurodegenerative disease, particularly a disease involving tau lesion.

### (2) Screening using PRKX gene overexpression primary nerve cell incorporating PRKX gene

The present invention also provides a method of screening for a prophylactic or therapeutic substance for a neurodegenerative disease, comprising contacting a test substance with a primary nerve cell incorporating a PRKX gene and overexpressing the PRKX gene and detecting, in the cell, phosphorylation, insolubilization or aggregation of tau, neurite denaturation or cell death.

The primary nerve cell used for the screening method is a nerve cell isolated from a mammal (e.g., rat, mouse, hamster, rabbit, dog, monkey and the like) that expresses tau endogenously, and is not particularly limited as long as it permits, when a nucleic acid encoding PRKX or a partial peptide thereof is introduced, sufficient observation of phosphorylation, insolubilization and aggregation of tau, neurite denaturation and cell death to the degree efficacy of a test substance can be evaluated with improvement of the tau lesion as an index. Primary nerve cell can be prepared from various mammals by a method known per se.

As for a nucleic acid encoding PRKX or a partial peptide thereof, introduction thereof into a primary nerve cell and culture of the cell harboring the gene, the same materials and methods as for the cell overexpressing a PRKX gene and a tau gene in the above-mentioned (1) can be used. The primary nerve cell in which PRKX gene is introduced and expressed can be confirmed by, for example, general immunostaining method using an anti-PRKX antibody.

Moreover, contact of PRKX gene overexpression primary nerve cell with a test substance, and the measurement of phosphorylation, insolubilization and aggregation of tau in the cell can also be performed in the same manner as in the screening in the above-mentioned (1).

Examples of the screening method using neurite denaturation in a PRKX gene overexpression primary nerve cell as an index include a method comprising immobilizing the cell, staining neurite by a general immunostaining method using an antibody against microtubuleprotein β-tubulin or microtubule-related protein MAP2, and measuring the neurite length and the like. Examples of the screening method using cell death of PRKX gene overexpression primary nerve cell as an index include a method comprising immobilizing the cell, staining the nucleus with a dye such as DAPI and the like and counting the positive cells and the like.

In the above-mentioned screening method, when the neurite length significantly increased in the presence of a test substance as compared to that in the absence of the test substance, the test substance can be selected as a candidate prophylactic or therapeutic substance for a neurodegenerative disease, particularly a disease involving tau lesion. When the number of DAPI positive cells significantly increased in the presence of a test substance as compared to that in the absence of the test substance, the test substance can be selected as a candidate prophylactic or therapeutic substance for a neurodegenerative disease, particularly a disease involving tau lesion.

### (3) Screening using PRKX protein and tau protein

The present inventors got an idea that the method using phosphorylation, insolubilization or aggregation of tau as an index in the screening in the above-mentioned (1) and (2) may be applicable to the system using isolated PRKX protein and tau protein, and tried and confirmed that it effectively functions as a screening system. Therefore, the present invention also provides a method of screening for a prophylactic or therapeutic substance for a neurodegenerative disease, comprising contacting a test substance with a PRKX protein and a tau protein, and detecting phosphorylation, insolubilization or aggregation of tau.

As the PRKX protein and tau protein used for the screening method, PRKX or a partial peptide thereof and tau or a partial peptide thereof described in detail for the screening in the above-mentioned (1) can be mentioned. The protein or peptide can be obtained, for example, by introducing a nucleic acid encoding PRKX or a partial peptide thereof, or a nucleic acid encoding tau or a partial peptide thereof in the above-mentioned (1) in a suitable host cell, culturing the obtained transformed cell, and recovering the protein or peptide from the culture. Useful hosts include, in addition to the above-mentioned mammalian cells, a bacterium of the genus *Escherichia,* a bacterium of the genus *Bacillus,* yeast, an insect cell, an insect and the like. As an expression vector for introduction and expression of a nucleic acid encoding PRKX or a partial peptide thereof, or a nucleic acid encoding tau or a partial peptide thereof in the host cell, one containing a promoter suitable for each host can be appropriately selected and used. Gene transfer method can also be selected appropriately from methods known per se according to the kind of the host. The obtained transformed cell can be cultured in a known medium used for culture of each host cell under general culture conditions (temperature, pH, aeration, stirring and the like, culture time etc.).

PRKX or a partial peptide thereof and tau or a partial peptide thereof can be separated and purified from the culture obtained by culturing the aforementioned transformant according to a method known per se.
For example, when PRKX or a partial peptide thereof and tau or a partial peptide thereof are extracted from cultured fungus bodies or cells, a method that comprises suspending the fungus bodies or cells collected from the culture by a known method in an appropriate buffer, disrupting the fungus bodies or cells by ultrasonication, lysozyme and/or freeze-thawing and the like, and performing centrifugation and filtration, to yield a crude extract of the soluble protein, and the like can be used as appropriate. The buffer may contain a protein denaturant such as urea or guanidine hydrochloride and a surfactant such as Triton X-100^{™}.

Isolation and purification of PRKX or a partial peptide thereof and tau or a partial peptide thereof contained in the thus-obtained soluble fraction can be conducted according to a method know *per se.* Useful methods include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like. These methods can be combined as appropriate.

Alternatively, PRKX protein and tau protein can also be isolated from cells or tissues producing them by a combination of protein separation and purification techniques similar to those mentioned above. In addition, a partial peptide of the protein can also be obtained by partial degradation of the obtained PRKX protein or tau protein by using a suitable peptidase and the like.

Moreover, PRKX or a partial peptide thereof and tau or a partial peptide thereof can also be produced based on the information of the amino acid sequence of the aforementioned protein and according to a publicly known method of peptide synthesis.
The method of peptide synthesis may be any of, for example, a solid phase synthesis process and a liquid phase synthesis process. A desired protein or peptide can be produced by condensing a partial peptide or amino acid capable of constituting the PRKX protein and tau protein with the remaining portion, and removing any protecting group the resultant product may have.
Here, the condensation and the removal of the protecting group are conducted according to methods known *per se,* for example, methods described in [1] to [5] below.
1) M. Bodanszky and M.A. Ondetti: Peptide Synthesis, Interscience Publishers, New York (1966)
2) Schroeder and Luebke: The Peptide, Academic Press, New York (1965)
3) Nobuo Izumiya, et al.: Peptide Gosei-no-Kiso to Jikken, published by Maruzen Co. (1975);
4) Haruaki Yajima and Shunpei Sakakibara: Seikagaku Jikken Koza 1, Tanpakushitsu no Kagaku IV, 205 (1977)
5) Haruaki Yajima, ed.: Zoku Iyakuhin no Kaihatsu, Vol. 14, Peptide Synthesis, published by Hirokawa Shoten.

PRKX protein and tau protein are each dissolved in water or aqueous solution (e.g., HEPES buffer, phosphate buffer, phosphoric acid buffered saline, Tris-HCl buffer, borate buffer, acetate buffer and the like) to a suitable concentration. For example, 200 ng/mL - 10 µg/mL can be mentioned as a concentration of PRKX, and 10 - 40 µg/mL can be mentioned as a concentration of tau.
A test substance can be contacted with the above-mentioned PRKX and tau solution by adding the test substance to the solution and performing incubation for a given time. While the concentration of the test substance to be added varies depending on the compound kind (solubility, toxicity etc.), for example, it is appropriately selected from the range of about 0.1 nM - about 100 nM. The incubate time is, for example, about 10 min - about 24 hr.

When the phosphorylation of tau and insolubilization of tau are used as indices, measurement and evaluation can be performed in the same manner as in the screening in the above-mentioned (1). When the aggregation of tau is used as an index, for example, measurement and evaluation can be performed by spotting the reaction mixture on a carrier, and adding a probe capable of recognizing a β sheet structure to the carrier for visualization.

Since a test substance selected by any of the screening methods of the above-mentioned (1) to (3) can suppress development and progression of a tau lesion, it is useful as a candidate prophylactic or therapeutic substance of a neurodegenerative disease, particularly a disease involving a tau lesion. Examples of the disease involving a tau lesion include, but are not limited to, Alzheimer's disease, Pick disease, corticobasal degeneration, progressive supranuclear palsy, frontotemporal dementia (FTDP-17) and the like. The in vivo prophylactic or therapeutic activity of a candidate substance of a prophylactic or therapeutic substance for a neurodegenerative disease obtained by the screening method of the present invention can be confirmed by, for example, administering the substance to an animal model (e.g., mutant tau (P301, LP301S, R406W and the like) Tg mouse, triple Tg mouse, r4510Tg mouse, wild-type tau Tg rat) showing a phenotype of abnormal accumulation of tau, and testing the improvement in the tau lesion.
The method of the present invention is particularly useful for screening for a prophylactic or therapeutic substance for Alzheimer's disease.

The thus-obtained a prophylactic or therapeutic substance for a neurodegenerative disease can be formulated by a conventional means.
The above-mentioned substance or a salt thereof itself may be administered, or administered in the form of a suitable pharmaceutical composition. A pharmaceutical composition used for the administration may contain the above-mentioned substance or a salt thereof and a pharmacologically acceptable carrier, diluent or excipient. Such pharmaceutical composition is provided in a dosage form suitable for oral or parenteral administration.

As a composition for parenteral administration, for example, injection, suppository and the like are used. Injection may comprise a dosage form such as intravenous injection, subcutaneous injection, intradermal injection, intramuscular injection, drip injection and the like. Such injection can be prepared according to a known method. A preparation method of injection include, for example, dissolving, suspending or emulsifying the above-mentioned substance or a salt thereof in an aseptic aqueous solution or oily solution generally used for injection. As an aqueous solution for injection, for example, an isotonic solution containing saline, glucose and other auxiliary agents and the like are used. It may be concurrently used with a suitable solubilizing agent such as alcohol (e.g., ethanol), polyalcohol (e.g., propylene glycol, polyethylene glycol), nonionic surfactant [e.g., polysorbate 80, HCO-50 (polyoxyethylene (50 mol) adduct of hydrogenated castor oil)] and the like. As an oily solution, for example, sesame oil, soybean oil and the like are used. It may be concurrently used with a solubilizing agent such as benzyl benzoate, benzyl alcohol and the like. A prepared injection is preferably filled in a suitable ampoule. A suppository to be used for rectal administration can also be prepared by mixing the above-mentioned substance or a salt thereof with a general base for suppository.

As a composition for oral administration, solid or liquid dosage form, specifically tablet (including sugar-coated tablet, film-coated tablet), pill, granule, powder, capsule (including soft capsule), syrup, emulsion, suspension and the like can be mentioned. Such composition is produced by a known method, and may contain a carrier, diluent or excipient generally used in the pharmaceutical field. As carrier or excipient for tablets, for example, lactose, starch, saccharose or magnesium stearate is used.

The above-mentioned parenteral or oral pharmaceutical composition is conveniently prepared in a dosage form of a dose unit suitable for the dose of an active ingredient. Examples of the dosage form for such dose unit include tablet, pill, capsule, injection (ampoule), and suppository. The above-mentioned substance or a salt thereof is preferably contained in an amount of generally 5 - 500 mg per dose unit dosage form, particularly 5 - 100 mg for injection and 10 - 250 mg for other dosage form.

While the dose of the above-mentioned medicament containing the above-mentioned substance or a salt thereof varies depending on the subject of administration, target disease, symptom, administration route and the like, the above-mentioned substance or a salt thereof is conveniently administered in, for example, generally about 0.01 - 20 mg/kg body weight, preferably about 0.1 - 10 mg/kg body weight, more preferably about 0.1 - 5 mg/kg body weight, as a single dose for the prophylaxis or treatment of Alzheimer's disease, which is administered about 1 to 5 times, preferably about 1 to 3 times, per day by intravenous injection. In other parenteral administration and oral administration, the amounts similar thereto can be administered. When the symptom is particularly severe, the dose may be increased depending on the symptom.

Abbreviations for bases, amino acids and the like used in the present specification are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine

The present invention is hereinafter described in more detail by means of the following Examples, which examples, however, are not to be construed as limiting the scope of the present invention.

### Examples

### Example 1

By PCR reaction using human brain-derived Marathon-Ready cDNA (Clontech) as a template and two primers, 5'-GTGCCCCGGACCTGAGTGC-3' (hPRKX-1F; SEQ ID NO: 5) and 5'-ACATTTTGCTTCTGGTCTGCT-3' (hPRKX-1R; SEQ ID NO: 6), human PRKX gene segment was amplified. The obtained DNA fragment was cloned. Human PRKX (hPRKXkai) amplified using the DNA fragment as a template and two primers 5'-CCTCGAGCCACCATGGAGGCGCCCGGGCTGGCCCAGGCGGCCGCGGCG-3 (change-1F; SEQ ID NO: 7) and hPRKX-1R were subcloned to the cloning site of a pTARGET expression vector (Promega) by TA cloning and nucleotide sequence was confirmed. The prepared human PRKX overexpression plasmid was named pPRKXkai. Inactive PRKX protein overexpression plasmid (pD172A) without kinase activity was prepared from pPRKXkai by substituting 172nd aspartic acid of PRKX protein to alanine by PCR reaction. That is, two PRKX partial DNA fragments with mutation were prepared from pPRKXkai by PCR using change-1F and 5'-GTTCTCTGGCTTCAAGGCCCTGTAGACG-3' (D172A-1R; SEQ ID NO: 8), 5'-CGTCTACAGGGCCTTGAAGCCAGAGAAC-3' (D172A-1F; SEQ ID NO: 9) and hPRKX-1R, and the obtained fragments was ligated by performing PCR reaction again using change-1F and hPRKX-1R as primers to give mutant PRKX full-length DNA. The fragment was cloned to the TA cloning site of a pTARGE expression vector.

### Example 2

pFB-Neo-CMV-tau plasmid obtained by inserting a DNA fragment containing a wild-type human tau gene bound at the downstream of a CMV promoter into a multicloning site of pFB-neo vector (Stratagene) was transferred into H4 neuroglial cells according to the instructions of ViraPort(R) Retroviral Gene Expression System (Stratagene), and a cell confirmed to show expression of tau was used as a H4-tau cell. The H4-tau cell was preserved at -80°C using Cellbanker-1 (JUJI FIELD INC.). pPRKXkai, pD172A and pTARGET vector as a control were each transferred into a H4-tau cell cultured overnight in DMEM medium (Invitrogen) containing 10% FBS (Invitrogen), according to the instructions of Fugene6 transfection reagent (Roche Diagnostics). One day later, the cells were dissolved in an SDS sample buffer (Daiichi Chemical), and the protein was separated using NuPAGE 4-12% Bis-Tris gel (Invitrogen). After transcription to a PVDF membrane, Western blot was performed using, as a primary antibody, an anti-S214 phosphorylated tau antibody (BioSource) for the detection of S214 phosphorylated tau, an Ab-3 antibody (Lab Vision) for the detection of total tau, and human PRKX for the detection of anti-PRKX antibody Abjent, and according to the instructions of ECL Advance Western Blotting Detection Kit (Amarsham Bioscience), and each protein was detected by LAS-1000plus lumino image analyzer (Fujifilm). As a result, 1.3-fold, 1.9-fold and 2.9-fold enhanced S214 phosphorylation was found in the cells incorporating 0.02 µg, 0.06 µg and 0.2 µg of PRKX expression plasmid, respectively. On the other hand, enhanced S214 phosphorylation was not found in pD172A tranfected cell. In addition, enhanced phosphorylation was not found in a cell added with 10 µM H-89 (BSO BioSource international) 2 hr before cell recovery. The tranfected cell was dissolved in a cell lysis buffer (BioSource), the amount of S214 phosphorylated tau was measured using a human pS214 tau ELISA kit (BioSource), the amount of total tau was measured using a human total tau ELISA kit (BioSource), each according to the instructions, and the amount of phosphorylated tau in the total tau was examined. As a result, 2.5-fold, 3.4-fold and 5.8-fold enhanced S214 phosphorylation was found in the cells incorporating 0.02 µg, 0.06 µg and 0.2 µg of PRKX expression plasmid, respectively. On the other hand, enhanced S214 phosphorylation was not found in the cell incorporating pD172A.

### Example 3

pPRKXkai and pTARGET vectors were each transferred into H4-tau cells cultured overnight in a DMEM medium containing 10% FBS, using a Fugene6 transfection reagent. One day later, H-89 was added at a concentration of 10 - 0.1 µM. 2 hr later, the cells were dissolved in a cell lysis buffer, the amount of S214 phosphorylated tau was measured using a human pS214 tau ELISA kit, the amount of the total tau was measured using a human total tau ELISA kit, and the amount of phosphorylated tau in the total tau was examined. As a result, the amount of tau S214 phosphorylation was suppressed at an IC50 value of 0.7 µM.

### Example 4

H4-tau cells were plated in a DMEM medium containing 10% FBS in a 96 well plate at a concentration of 1 x 10⁴ cells/well and, the next day, pPRKXkai and pTARGET vectors were each transferred thereinto using a Fugene6 transfection reagent. One day later, H-89 was added to the medium at a concentration of 10 - 0.1 µM. 2 hr later, the cells were fixed with 4% para-formaldehyde (Wako Pure Chemical Industries, Ltd.), washed with phosphate buffer, and blocked with phosphate buffer containing 1.5% BSA (Sigma Aldrich) and 0.1% Triton X-100 (Wako Pure Chemical Industries, Ltd.). Thereafter, as a primary antibody, an anti-S214 phosphorylated tau antibody was used to label phosphorylated tau and an anti-tau monoclonal antibody HT-7 (Pierce Biotechnology) was used to label total tau. After washing, they were reacted with an anti-rabbit Alexa Fluor 680-labeled secondary antibody (Invitrogen) and an anti-mouse IRD800CW-labeled secondary antibody (Wako Pure Chemical Industries, Ltd.), as secondary antibodies. After washing, the amounts of S214 phosphorylated tau and total tau were detected using 2 fluorescence system infrared fluorescence Imager Odyssey (ALOKA CO., LTD.). As a result, the amount of tau S214 phosphorylation was suppressed at an IC50 value of 0.7 µM.

### Example 5

hPRKXkai was cleaved from pPRKXkai with restriction enzymes Xho I and Not I, and inserted in between Xho I and Not I of lentivirus vector CSII-CMV-MCB (RIKEN, Japan) to give a lentivirus plasmid (hPRKXonlenti) for PRKX expression. hPRKXonlenti was transferred together with packaging plasmid (pCAG-HIVgp), VSV-G and Rev expression plasmid (pCMV-VSV-G-RSV-Rev) into HEK-293T cells cultured overnight in a DMEM medium supplemented with 10% FBS. After 18 hr, the medium was removed, a DMEM medium supplemented with 10% FBS and forskolin (10 µm, Wako Pure Chemical Industries, Ltd.) was added, and the cells were cultured for 48 hr. The supernatant was collected, filtered through a 0.45 µM filter and the filtrate was centrifuged at 20°C, 50000 rpm for 2 hr. After centrifugation, the precipitate was collected and dissolved in an HBSS buffer (Invitrogen). Using HIV-1 p24 ELISA kit (Perkin Elmer Inc.), the titer was measured with 1 ng of p24 as 10000u. The produced virus was preserved at -80°C. Tau overexpression human neuroblast SHSY-5Y was infected with the prepared PRKX overexpression lentivirus and, as a control, lacZ overexpression lentivirus at MOI=1. Three days later, the cells were dissolved in an SDS sample buffer and the protein was collected. Simultaneously, virus uninfected cells were also collected. These proteins were separated by using NuPAGE 4-12% Bis-Tris gel and transcribed to a PVDF membrane. Western blot was performed using, as a primary antibody, an anti-S214 phosphorylated tau antibody for the detection of S214 phosphorylated tau, an Ab-3 antibody for the detection of total tau, and an anti-PRKX antibody for the detection of human PRKX, and ECL Advance Western Blotting Detection Kit, and each protein was detected by LAS-1000plus luminoimage analyzer. As a result, enhanced S214 phosphorylation was found in the PRKX infection cells.

### Example 6

Rat primary nerve cells (DIV13) plated in a 48 well plate at a concentration of 3.5 x 10³ cells/well were infected with PRKX overexpression lentivirus (MOI=1, 3, 6), lacZ overexpression lentivirus (MOI=6), and further, D172A overexpression lentivirus (MOI=1, 6) produced in the same manner. Six days later, each was dissolved in a cell lysis buffer. Tau protein was trapped on a capture plate in the mouse total tau ELISA kit (BioSource), and the amount of S214 phosphorylated tau in the solution was measured using the detection reagent in the human pS214 tau ELISA kit. As a standard solution used for producing a standard curve, a cell lysate of the rat primary nerve cell stimulated with forskolin for 1 hr to enhance S214 phosphorylation was used. The amount of total tau was measured using a mouse total tau ELISA kit (BioSource) and according to the instructions thereof. As the standard solution, a cell lysate of the above-mentioned rat primary nerve cells stimulated with forskolin for 1 hr was used. As a result, 3.0-fold, 4.2-fold and 6.3-fold enhanced amounts of S214 phosphorylated tau were found in the cells infected with PRKX overexpression lentivirus at M01=1, 3 and 6, respectively. On the other hand, D172A overexpression lentivirus and lacZ overexpression lentivirus did not show an enhanced amount of S214 phosphorylated tau.

### Example 7

DIV14 rat primary nerve cells plated in a 48 well plate at a concentration of 3.5 x 10³ cells/well were each infected with PRKX, lacZ and D172A overexpression lentiviruses at MOI=2. Ten days later, the cells were dissolved in an SDS sample buffer, and proteins were collected. The proteins were separated using NuPAGE 4-12% Bis-Tris gel and transferred to a PVDF membrane. Western blot was performed using, as a primary antibody, an anti-S214 phosphorylated tau antibody for the detection of S214 phosphorylated tau, and an Ab-3 antibody for the detection of total tau, and according to the instructions of ECL Advance Western Blotting Detection Kit, and each protein was detected by LAS-1000plus luminoimage analyzer. As a result, enhanced S214 phosphorylation was found in PRKX expression cells, and was not found in D172A overexpression lentivirus and lacZ overexpression lentivirus.

### Example 8

DIV13 rat primary nerve cells plated in a 48 well plate at a concentration of 3.5 x 10³ cells/well were infected with produced PRKX, lacZ and D172A overexpression lentiviruses at MOI=3. Seven days later, the cells were fixed with 4% para-formaldehyde, washed with phosphate buffer, and blocked with a phosphate buffer containing 1.5% BSA and 0.1% Triton X-100. Thereafter, as a primary antibody, an anti-S214 phosphorylated tau antibody was used to label S214 phosphorylated tau. After washing with phosphate buffer, the S214 phosphorylated tau was reacted with an anti-rabbit Alexa Fluor 594-labeled secondary antibody (Invitrogen) as a secondary antibody for 30 min. After washing with phosphate buffer, the nucleus was stained with DAPI (Molecular Probe). The stained S214 phosphorylated tau and the nucleus were photographed using Discovery-1 (Molecular Device). As a result, enhanced amount of S214 phosphorylated tau was found in PRKX infection cells, and was not found in the D172A overexpression lentivirus.

### Example 9

DIV12 mouse primary nerve cells plated in a 48 well plate at a concentration of 3.5 x 10³ cells/well were infected with PRKX overexpression lentiviruses at MOI=2. Two days later, the cells were fixed with 4% para-formaldehyde, washed with phosphate buffer, and blocked with a phosphate buffer containing 1.5% BSA and 0.1% Triton X-100. Thereafter, as a primary antibody, an anti-S214 phosphorylated tau antibody was used to label phosphorylated tau, and an anti-MAP2 monoclonal antibody (Chemicon International) was used to label nerve cells. After washing, anti-S214 phosphorylated tau antibody was stained with anti-rabbit alexafluoro594-labeled secondary antibody, and anti-MAP2 antibody was stained with anti-mouse alexafluoro488-labeled secondary antibody (Invitrogen), and they were photographed using Discovery-1. As a result, the amount of tau S214 phosphorylation decreased due to H-89.

### Example 10

H4-tau cells were plated in a 6 well plate at a concentration of 1 x 10⁵ cells/well and, the next day, infected with PRKX or lacZ overexpression lentivirus at MOI=1. Five days later, the cells were washed with phosphate buffer, collected in a high salt concentration-RAB buffer (0.1M 2-(Nmorpholino)-ethanesulfonic acid, 0.75M NaCl, 0.5 mM MgSO4, 1 mM Phenylmethyl sulfonyl fluoride (PMSF), 0.1 mM EDTA, 1 mM EGTA, 0.02M NaF, 1mM Na₃VO₄ and Protease inhibitor cocktail (Roche Diagnostics), pH 7.2), disrupted with 22G injection needle and 1 ml injection syringe, and left standing for 20 min on ice. Thereafter, the cells were centrifuged (20,000 x g, 20 min.), and the obtained supernatant was collected as an RAB soluble fraction. The precipitate was washed three times with the high salt concentration-RAB buffer, and dissolved in RIPA buffer (10 mM Tris-HCl pH 8.0, 140 mM NaCl, 1% NP-40, 0.5% sodium deoxycholate, 1 mM Iodoacetamide, 0.1% SDS, 0.02M NaF, 1 mM Na₃VO₄, 1 mM PMSF and Protease inhibitor cocktail). After disrupting in an ultrasonicator, the cells were centrifuged for 20 min at 20,000 x g. The centrifuge was washed three times with RIPA buffer, and the precipitate was dissolved in an SDS sample buffer to give an RIPA insoluble fraction. The protein concentration in the sample buffer was measured using an EZQ protein quantification kit (Invitrogen) and BSA as a standard. The proteins (each 2 µg) in the obtained RIPA insoluble fraction were migrated and separated in NuPAGE 4-12% Bis-Tris gel. After transcription to a PVDF membrane, Western blot was performed using an anti-total tau antibody, and detergent insoluble tau (RIPA insoluble tau) was detected. The band of the tau obtained by Western blot was quantified. It was shown that the band concentration of tau increased 1.8-fold in PRKX-infected cells as compared to LacZ infected cells, which indicates increase of detergent-insoluble tau. In PRKX infected cells, moreover, a thin band was observed at a position assumed to be dimer and trimer of tau, and smear staining of aggregated tau was also found on the polymer side.

### Example 11

H4-tau cells were plated in a 24 well plate at a concentration of 1 x 10⁵ cells/well and, the next day, infected with PRKX or lacZ overexpression lentivirus at MOI=1 or 2.5. Three days later, H-89 was added to a concentration of 3 µM. The next day, the cells fixed with 4% para-formaldehyde were washed with phosphate buffer, and blocked with phosphate buffer containing 1.5% BSA and 0.1% Triton X-100. Thereafter, the cells were stained by using S214 phosphorylated tau, and anti-S214 phosphorylated tau or total tau antibody as a primary antibody and anti-rabbit Alexa Fluor 594-labeled secondary antibody as a secondary antibody. Thereafter, the cells were left standing in 0.005% aqueous thioflavin S (Sigma Ltd.) solution for 5 min, and washed twice with 70% ethanol and once with water. Furthermore, the nucleus was stained with DAPI. The stained cells were photographed by Discovery-1, and the number of DAPI positive cells and the number of thioflavin S positive cells were counted by metamorph software (Molecular Device) based on the images taken by Discovery-1. In the PRKX infected well, the number of cells stained with thioflavin S having a β sheet structure increased 2-fold at MOI=1 and 5.2-fold at MOI=2.5 as compared to lacZ infected well. By costaining with a tau specific antibody, almost all thioflavin S positive images were costained with tau and S214 phosphorylated tau. In addition, increase of thioflavin S stained cells, which was found by infection at MOI=2.5, was suppressed by 75% by the addition of 3 µM H-89.

### Example 12

H4 cells were plated in a 48 well plate at a concentration of 3.5 x 10³ cells/well and, the next day, infected with PRKX or lacZ overexpression lentivirus at MOI=1. Two days later, H-89 was added at 3 or 10 µM. The next day, the cells were fixed, and the nucleus was stained with DAPI. DAPI positive cells were photographed by Discovery-1, and the number of DAPI positive cells was counted from the images by a Metamorph software. As a result, PRKX expression cells were found to have decreased by 59%. The cell death was suppressed by 29% or 46% by the addition of 3 µM or 10 µM H-89.

### Example 13

DIV14 rat primary nerve cells were plated in a 48 well plate at a concentration of 3.5 x 10³ cells/well and infected with PRKX or lacZ overexpression lentivirus at MOI=2. Six days after the infection, the cells were fixed and, as a primary antibody, anti-PRKX antibody was used to label PRKX, and anti-MAP2 monoclonal antibody (Chemicon International) was used to label nerve cell. An anti-PRKX antibody was stained with anti-rabbit Alexa Fluor 594-labeled secondary antibody, anti-MAP2 antibody was stained with anti-mouse Alexa Fluor 488-labeled secondary antibody, the nucleus was stained with DAPI, and they were photographed by Discovery-1. The cell number and neurite elongation were measured by a metamorph software based on the images taken by Discovery-1. As a result, it was found that the neurite elongation stained with MAP2 decreased by 37% in PRKX overexpression cells, and denaturation of neurite was induced. Furthermore, the number of MAP2 positive cells also decreased by 26%.

### Example 14

DIV14 rat primary nerve cells were plated in a 48 well plate at a concentration of 3.5 x 10³ cells/well and infected with PRKX overexpression lentivirus at MOI=1. Four days after the infection, forskolin was added at a concentration of 10 µM, and the cells were cultured for 2 days. The cells were fixed and, as a primary antibody, anti-PRKX antibody was used to label PRKX, and anti-MAP2 monoclonal antibody was used to label nerve cell. The anti-PRKX antibody was stained with anti-rabbit Alexa Fluor594-labeled secondary antibody, anti-MAP2 antibody was stained with anti-mouse Alexa Fluor488-labeled secondary antibody, the nucleus was stained with DAPI, and they were photographed by Discovery-1. As a result, neurodegeneration by PRKX expression was accelerated by forskolin.

### Example 15

DIV14 mouse primary nerve cells were plated in a 48 well plate at a concentration of 3.5 x 10³ cells/well and infected with PRKX or D172A overexpression lentivirus at MOI=2. Four days after the infection, forskolin was added at a concentration of 10 µM, and the cells were cultured for 2 days. The cells were fixed, nerve cell was labeled with anti-MAP2 monoclonal antibody as a primary antibody, anti-MAP2 antibody was stained with anti-mouse Alexa Fluor 488-labeled secondary antibody, and they were photographed by Discovery-1. The neurite elongation was measured by a metamorph software based on the images taken by Discovery-1. As a result, the decrease of the neurite elongation found with the PRKX overexpression lentivirus was suppressed by 76% by H-89.

### Example 16

To a reaction mixture (40 mM Tris-HCl (pH 7.5), 20 mM MgCl₂, 0.1 mg/ml BSA, 25 µl) containing recombinant PRKX protein (Invitrogen, 4 µg/ml) and recombinant tau protein (Wako Pure Chemical Industries, Ltd., 40 µg/ml) was added H-89 solution (40 mM Tris-HCl (pH 7.5), 0.1% DMSO, 20 mM MgCl₂, 0.1 mg/ml BSA, 5 µl) to a concentration during reaction of 1 or 0.1 µM. As a control, 40 mM Tris-HCl (pH 7.5), 0.1% DMSO, 20 mM MgCl₂, 0.1 mg/ml BSA solution (5 µl) were added. 25 µM ATP solution (40 mM Tris-HCl (pH 7.5), 20 mM MgCl₂, 0.1mg/ml BSA) was added by 20 µl to start the reaction and the mixture was left standing at 30°C for 60 min. An SDS sample buffer was added to quench the reaction, the protein was separated using NuPAGE 4-12% Bis-Tris gel and transferred to a PVDF membrane. Western blot was performed using an anti-S214 phosphorylated tau antibody as a primary antibody and ECL Advance Western Blotting Detection Kit, and S214 phosphorylated tau was quantified by LAS-1000plus lumino image analyzer. It was found that the amount of S214 phosphorylation was enhanced by the addition of ATP as compared to the control without addition of ATP. By the addition of H-89 at a final concentration of 1 and 0.1 µM, the tau S214 phosphorylation reaction decreased to 5% and 52%, respectively, of that without the addition.

### Industrial Applicability

Since the screening system of the present invention enables conventionally unavailable search and evaluation of a prophylactic or therapeutic drug for a neurodegenerative disease using a tau lesion improvement effect as an index, a promising candidate substance of a prophylactic or therapeutic drug for neurodegenerative diseases can be screened for more efficiently.

This application is based on patent application No. 2008-030946 filed in Japan, the contents of which are encompassed in full herein.

## Claims

1. A method of screening for a prophylactic or therapeutic substance for a neurodegenerative disease, comprising contacting a test substance with a cell overexpressing a PRKX gene and a tau gene, which incorporates the PRKX and tau genes, and detecting phosphorylation, insolubilization or aggregation of tau in the cell.

2. The method according to claim 1, wherein the phosphorylation of tau is phosphorylation of the 214-position Ser.

3. The method according to claim 1, further comprising measuring the amount of detergent-insoluble tau.

4. The method according to claim 1, further comprising counting cells positive to a probe recognizing α β sheet structure.

5. The method according to claim 1, wherein the cell is an established cell line.

6. The method according to claim 1, wherein the cell is a H4 neuroglial cell.

7. A method of screening for a prophylactic or therapeutic substance for a neurodegenerative disease, comprising contacting a test substance with a primary nerve cell incorporating a PRKX gene and overexpressing the PRKX gene and detecting, in the cell, phosphorylation, insolubilization or aggregation of tau, neurite denaturation or cell death,

8. The method according to claim 1 or 7, wherein the PRKX gene is introduced by a virus vector.

9. The method according to claim 8, wherein the virus vector is lentivirus.

10. A method of screening for a prophylactic or therapeutic substance for a neurodegenerative disease, comprising contacting a test substance with a PRKX protein and a tau protein, and detecting phosphorylation, insolubilization or aggregation of tau.

11. The method according to claim 1, 7 or 10, wherein the neurodegenerative disease is a disease involving a tau lesion.

12. The method according to claim 11, wherein the disease involving a tau lesion is Alzheimer's disease.


SEQUENCE LISTING

<110>  TAKEDA PHARMACEUTICAL COMPANY LIMITED

<120>  Screening Method

<130>  091318

<150>  JP2008-030946
<151>  2008-02-12

<160>  9

<170>  PatentIn version 3.5

<210>  1
<211>  1074
<212>  DNA
<213>  Homo sapiens


<220>
<221>  CDS
<222>  (1)..(1074)

<400>  1
atg gag gcg ccc ggg ctg gcc cag gcg gcc gcg gcg gag agc gac tcc       48
Met Glu Ala Pro Gly Leu Ala Gln Ala Ala Ala Ala Glu Ser Asp Ser
1               5                   10                  15

cgc aag gtg gcg gag gag acc ccc gac ggg gcg ccc gcg ctc tgc ccc       96
Arg Lys Val Ala Glu Glu Thr Pro Asp Gly Ala Pro Ala Leu Cys Pro
            20                  25                  30

agc cct gag gcg ctg tcg ccg gag ccg cct gtg tac agc ctg cag gac      144
Ser Pro Glu Ala Leu Ser Pro Glu Pro Pro Val Tyr Ser Leu Gln Asp
        35                  40                  45

ttt gac acg ctg gcc acc gtg ggc act ggg acg ttc ggg cgg gtg cac      192
Phe Asp Thr Leu Ala Thr Val Gly Thr Gly Thr Phe Gly Arg Val His
    50                  55                  60

ctg gtg aag gag aag aca gcc aag cat ttc ttc gcc ctc aag gtg atg      240
Leu Val Lys Glu Lys Thr Ala Lys His Phe Phe Ala Leu Lys Val Met
65                  70                  75                  80

agc att ccc gac gtc atc cgc cta aag cag gag caa cac gta cac aat      288
Ser Ile Pro Asp Val Ile Arg Leu Lys Gln Glu Gln His Val His Asn
                85                  90                  95

gag aag tct gtc ctg aag gaa gtc agc cac ccg ttc ctc atc agg ctg      336
Glu Lys Ser Val Leu Lys Glu Val Ser His Pro Phe Leu Ile Arg Leu
            100                 105                 110

ttc tgg acg tgg cat gac gag cgc ttc ctc tac atg ctc atg gag tac      384
Phe Trp Thr Trp His Asp Glu Arg Phe Leu Tyr Met Leu Met Glu Tyr
        115                 120                 125

gtg ccg ggc ggc gag ctc ttc agc tac ctg cgc aac cgg ggg cgc ttc      432
Val Pro Gly Gly Glu Leu Phe Ser Tyr Leu Arg Asn Arg Gly Arg Phe

130                 135                 140

tcc agc acc acg ggg ctc ttc tac tct gca gag atc atc tgt gcc atc      480
Ser Ser Thr Thr Gly Leu Phe Tyr Ser Ala Glu Ile Ile Cys Ala Ile
145                 150                 155                 160

gag tac ctg cac tcc aaa gag atc gtc tac agg gac ttg aag cca gag      528
Glu Tyr Leu His Ser Lys Glu Ile Val Tyr Arg Asp Leu Lys Pro Glu
                165                 170                 175

aac atc ctg ctg gat agg gat ggc cac att aag ctc acg gac ttt ggg      576
Asn Ile Leu Leu Asp Arg Asp Gly His Ile Lys Leu Thr Asp Phe Gly
            180                 185                 190

ttc gcc aag aag ctg gta gac agg act tgg acc ctc tgt gga aca ccc      624
Phe Ala Lys Lys Leu Val Asp Arg Thr Trp Thr Leu Cys Gly Thr Pro
        195                 200                 205

gag tac cta gcc ccc gaa gtc att cag agc aag ggc cac gga agg gcc      672
Glu Tyr Leu Ala Pro Glu Val Ile Gln Ser Lys Gly His Gly Arg Ala
    210                 215                 220

gtg gac tgg tgg gcc ctc ggc atc ctg ata ttc gag atg ctt tcg ggg      720
Val Asp Trp Trp Ala Leu Gly Ile Leu Ile Phe Glu Met Leu Ser Gly
225                 230                 235                 240

ttt cct ccg ttt ttt gat gac aac ccg ttt ggc att tat cag aaa att      768
Phe Pro Pro Phe Phe Asp Asp Asn Pro Phe Gly Ile Tyr Gln Lys Ile
                245                 250                 255

ctt gca ggc aaa ata gat ttc ccc aga cat ttg gat ttc cat gta aaa      816
Leu Ala Gly Lys Ile Asp Phe Pro Arg His Leu Asp Phe His Val Lys
            260                 265                 270

gac ctc att aag aaa ctg ctc gtg gtt gac aga aca agg cga tta gga      864
Asp Leu Ile Lys Lys Leu Leu Val Val Asp Arg Thr Arg Arg Leu Gly
        275                 280                 285

aac atg aag aac ggg gcg aat gat gtg aag cat cat cgg tgg ttc cgc      912
Asn Met Lys Asn Gly Ala Asn Asp Val Lys His His Arg Trp Phe Arg
    290                 295                 300

tcc gtg gac tgg gaa gct gtt ccg cag aga aaa ctg aag cct ccc atc      960
Ser Val Asp Trp Glu Ala Val Pro Gln Arg Lys Leu Lys Pro Pro Ile
305                 310                 315                 320

gtg ccc aag ata gct ggt gac ggc gac act tcc aac ttc gaa act tac     1008
Val Pro Lys Ile Ala Gly Asp Gly Asp Thr Ser Asn Phe Glu Thr Tyr
                325                 330                 335

cct gag aat gac tgg gac aca gcc gcg ccc gtg ccg cag aag gat tta     1056
Pro Glu Asn Asp Trp Asp Thr Ala Ala Pro Val Pro Gln Lys Asp Leu
            340                 345                 350

gaa atc ttc aag aat ttc                                             1074
Glu Ile Phe Lys Asn Phe
        355

<210>  2

<211>  358
<212>  PRT
<213>  Homo sapiens

<400>  2
   Met Glu Ala Pro Gly Leu Ala Gln Ala Ala Ala Ala Glu Ser Asp Ser
   1               5                   10                  15
   Arg Lys Val Ala Glu Glu Thr Pro Asp Gly Ala Pro Ala Leu Cys Pro
               20                  25                  30
   Ser Pro Glu Ala Leu Ser Pro Glu Pro Pro Val Tyr Ser Leu Gln Asp
           35                  40                  45
   Phe Asp Thr Leu Ala Thr Val Gly Thr Gly Thr Phe Gly Arg Val His
       50                  55                  60
   Leu Val Lys Glu Lys Thr Ala Lys His Phe Phe Ala Leu Lys Val Met
   65                  70                  75                  80
   Ser Ile Pro Asp Val Ile Arg Leu Lys Gln Glu Gln His Val His Asn
                   85                  90                  95
   Glu Lys Ser Val Leu Lys Glu Val Ser His Pro Phe Leu Ile Arg Leu
               100                 105                 110
   Phe Trp Thr Trp His Asp Glu Arg Phe Leu Tyr Met Leu Met Glu Tyr
           115                 120                 125
   Val Pro Gly Gly Glu Leu Phe Ser Tyr Leu Arg Asn Arg Gly Arg Phe
       130                 135                 140
   Ser Ser Thr Thr Gly Leu Phe Tyr Ser Ala Glu Ile Ile Cys Ala Ile
   145                 150                 155                 160
   Glu Tyr Leu His Ser Lys Glu Ile Val Tyr Arg Asp Leu Lys Pro Glu
                   165                 170                 175
   Asn Ile Leu Leu Asp Arg Asp Gly His Ile Lys Leu Thr Asp Phe Gly
               180                 185                 190
   Phe Ala Lys Lys Leu Val Asp Arg Thr Trp Thr Leu Cys Gly Thr Pro
           195                 200                 205
   Glu Tyr Leu Ala Pro Glu Val Ile Gln Ser Lys Gly His Gly Arg Ala
       210                 215                 220
   Val Asp Trp Trp Ala Leu Gly Ile Leu Ile Phe Glu Met Leu Ser Gly
   225                 230                 235                 240
   Phe Pro Pro Phe Phe Asp Asp Asn Pro Phe Gly Ile Tyr Gln Lys Ile
                   245                 250                 255
   Leu Ala Gly Lys Ile Asp Phe Pro Arg His Leu Asp Phe His Val Lys
               260                 265                 270
   Asp Leu Ile Lys Lys Leu Leu Val Val Asp Arg Thr Arg Arg Leu Gly
           275                 280                 285
   Asn Met Lys Asn Gly Ala Asn Asp Val Lys His His Arg Trp Phe Arg
       290                 295                 300
   Ser Val Asp Trp Glu Ala Val Pro Gln Arg Lys Leu Lys Pro Pro Ile
   305                 310                 315                 320
   Val Pro Lys Ile Ala Gly Asp Gly Asp Thr Ser Asn Phe Glu Thr Tyr
                   325                 330                 335
   Pro Glu Asn Asp Trp Asp Thr Ala Ala Pro Val Pro Gln Lys Asp Leu
               340                 345                 350
   Glu Ile Phe Lys Asn Phe
           355

<210>  3
<211>  1323
<212>  DNA
<213>  Homo sapiens

<220>
<221>  CDS
<222>  (1)..(1323)

<400>  3
atg gct gag ccc cgc cag gag ttc gaa gtg atg gaa gat cac gct ggg       48
acg tac ggg ttg ggg gac agg aaa gat cag ggg ggc tac acc atg cac       96
caa gac caa gag ggt gac acg gac gct ggc ctg aaa gaa tct ccc ctg      144
cag acc ccc act gag gac gga tct gag gaa ccg ggc tct gaa acc tct 192
gat gct aag agc act cca aca gcg gaa gat gtg aca gca ccc tta gtg 240
gat gag gga gct ccc ggc aag cag gct gcc gcg cag ccc cac acg gag 288
atc cca gaa gga acc aca gct gaa gaa gca ggc att gga gac acc ccc 336
agc ctg gaa gac gaa gct gct ggt cac gtg acc caa gct cgc atg gtc 384
agt aaa agc aaa gac ggg act gga agc gat gac aaa aaa gcc aag ggg 432
gct gat ggt aaa acg aag atc gcc aca ccg cgg gga gca gcc cct cca 480
ggc cag aag ggc cag gcc aac gcc acc agg att cca gca aaa acc ccg 528
ccc gct cca aag aca cca ccc agc tct ggt gaa cct cca aaa tca ggg 576
gat cgc agc ggc tac agc agc ccc ggc tcc cca ggc act ccc ggc agc 624
cgc tcc cgc acc ccg tcc ctt cca acc cca ccc acc cgg gag ccc aag 672
aag gtg gca gtg gtc cgt act cca ccc aag tcg ccg tct tcc gcc aag 720
agc cgc ctg cag aca gcc ccc gtg ccc atg cca gac ctg aag aat gtc 768
aag tcc aag atc ggc tcc act gag aac ctg aag cac cag ccg gga ggc 816
ggg aag gtg cag ata att aat aag aag ctg gat ctt agc aac gtc cag 864
tcc aag tgt ggc tca aag gat aat atc aaa cac gtc ccg gga ggc ggc     912
agt gtg caa ata gtc tac aaa cca gtt gac ctg agc aag gtg acc tcc     960
aag tgt ggc tca tta ggc aac atc cat cat aaa cca gga ggt ggc cag     1008
gtg gaa gta aaa tct gag aag ctt gac ttc aag gac aga gtc cag tcg     1056
aag att ggg tcc ctg gac aat atc acc cac gtc cct ggc gga gga aat     1104
aaa aag att gaa acc cac aag ctg acc ttc cgc gag aac gcc aaa gcc     1152
aag aca gac cac ggg gcg gag atc gtg tac aag tcg cca gtg gtg tct     1200
ggg gac acg tct cca cgg cat ctc agc aat gtc tcc tcc acc ggc agc     1248
atc gac atg gta gac tcg ccc cag ctc gcc acg cta gct gac gag gtg     1296
tct gcc tcc ctg gcc aag cag ggt ttg                                 1323


<210>  4
<211>  441
<212>  PRT
<213>  Homo sapiens

<400>  4

<210>  5
<211>  19
<212>  DNA
<213>  Artificial Sequence

<220>
<223>  Primer

<400>  5
gtgccccgga cctgagtgc                                                    19

<210>  6
<211>  21
<212>  DNA
<213>  Artificial Sequence

<220>
<223>  Primer

<400>  6
acattttgct tctggtctgc t                                                 21


<210>  7
<211>  48
<212>  DNA
<213>  Artificial Sequence

<220>
<223>  Primer

<400>  7
cctcgagcca ccatggaggc gcccgggctg gcccaggcgg ccgcggcg                    48


<210>  8
<211>  28
<212>  DNA
<213>  Artificial Sequence

<220>
<223>  Primer

<400>  8
gttctctggc ttcaaggccc tgtagacg                                          28


<210>  9
<211>  28
<212>  DNA
<213>  Artificial Sequence

<220>
<223>  Primer

<400>  9
cgtctacagg gccttgaagc cagagaac                                          28